# EUROPÄISCHE PATENTSCHRIFT

(11) **EP 3 619 219 B1**
(45) Veröffentlichungstag und Bekanntmachung des Hinweises auf die Patenterteilung: **01.11.2023**
(21) Anmeldenummer: 15753336.5
(22) Anmeldetag: 12.08.2015
(51) Int. Cl.: C07F 3/02, B01J 31/00, C08F 4/08

(54) **NIEDRIGVISKOSE LÖSUNGEN VON ERDALKALIMETALLALKOXIDEN IN APROTISCHEN LÖSUNGSMITTELN, VERFAHREN ZU DEREN HERSTELLUNG UND VERWENDUNG FÜR DIE HERSTELLUNG VON ZIEGLER NATTA - KATALYSATOREN**
LOW-VISCOSITY SOLUTIONS OF ALKALINE-EARTH METAL ALKOXIDES IN APROTIC SOLVENTS, METHOD FOR THE PRODUCTION OF SAME AND USE FOR THE PRODUCTION OF ZIEGLER-NATTA CATALYSTS
SOLUTIONS FAIBLEMENT VISQUEUSES D'ALCOOLATES MÉTALLIQUES ALCALINO-TERREUX DANS DES SOLVANTS APROTIQUES, PROCÉDÉ POUR LEUR FABRICATION ET UTILISATION POUR LA FABRICATION DE CATALYSEURS DE ZIEGLER-NATTA

(30) Priorität: 12.08.2014 DE 102014215919; 13.08.2014 DE 102014216067; 26.11.2014 DE 102014224139
(43) Veröffentlichungstag der Anmeldung: 11.03.2020
(73) Patentinhaber: Albemarle Germany GmbH, 65926 Frankfurt am Main (DE)
(72) Erfinder: WIETELMANN, Ulrich, 61381 Friedrichsdorf (DE); EMMEL, Ute, 65929 Frankfurt am Main (DE); STOLL, Armin, 69493 Hirschberg an der Bergstraße (DE); KIEFER, Florian, 85354 Freising (DE)
(74) Vertreter: Uexküll & Stolberg
(86) Internationale Anmeldenummer: PCT/EP2015/068513
(87) Internationale Veröffentlichungsnummer: WO 2016/023932

(56) Entgegenhaltungen:
- EP-A1- 0 027 577
- US-A- 3 903 019
- US-A- 4 133 824
- US-A- 4 634 786

## Beschreibung

Die Erfindung betrifft Lösungen von Erdalkalimetallalkoxiden in Mischung mit Erdalkalimetallalkylen sowie Verfahren zu deren Herstellung.

Magnesiumalkoxide werden u.a. zur Herstellung geträgerter Olefin-Polymerisationskatalysatoren vom Ziegler-Natta - Typ benötigt. Dazu werden gemäß Dokument EP 1031580 unlösliche Alkoxide wie z.B. Magnesiumethoxid in Form sphärischer Partikel eingesetzt, welche durch Reaktion mit Titanchlorid oder einer anderen Titan-Halogenbindungen aufweisenden Verbindung (z. B. Cp₂TiCl₂) in die aktive Form überführt:

Mg(OEt)₂ + Cp₂TiCl₂ → Mg(OEt)₂₋ₓClₓ + Cp₂TiCl₂₋ₓ(OEt)ₓ

(x = 0 bis 2)

Dokument WO 85/02176 beschreibt eine weitere Möglichkeit, geträgerte Ziegler-Natta - Katalysatoren herzustellen auszugehend von löslichen Magnesiumalkoxiden. Während die meisten Magnesiumalkoholate wie z.B. die Mg-Salze des Methanols, Ethanols, Propanols, Isopropanols, tert-Butanols etc in aprotischen Lösungsmitteln unlöslich sind, erweisen sich viele Mg-Verbindungen primärer Alkohole, die eine Verzweigung in 2-Stellung aufweisen, als löslich in Kohlenwasserstoffen. Aus der Schrift WO 85/02176 ist bekannt, dass sich beispielsweise die Magnesiumsalze des 2-Methyl-1-pentanols oder des 2-Ethyl-1-hexanols in Konzentrationen von 1,3 mol/l in Cyclohexan lösen. Auch gemischte Mg-alkoxide, d.h. solche mit zwei verschiedenen Alkoxid-Resten Mg(OR¹)ₙ(OR²)₂₋ₙ können kohlenwasserstofflöslich sein, wenn der korrespondierende Alkohol R¹OH ein in 2-Stellung verzweigter primärer Alkohol und der korrespondierende Alkohol R²OH beispielsweise ein sekundärer Alkohol ist.

Nachteilig an Kohlenwasserstoff-Lösungen, die außer Magnesium kein weiteres gelöstes Metall aufweisen, sind die relativ hohen Viskositäten. Weiterhin ist es nicht möglich, solche Lösungen direkt durch Umsetzung von Magnesiummetall mit dem Alkohol im gewünschten Kohlenwasserstoff herzustellen, ohne störende Hilfsmittel zuzugeben. Um eine direkte Umsetzung überhaupt zu ermöglichen, muß das Magnesiummetall aktiviert werden, was durch Anätzen mit Iod erreicht werden kann. Auch mit dieser Maßnahme ist die Umsetzungsgeschwindigkeit selbst beim Einsatz von hochreaktivem Mg-Pulver noch sehr gering. So wird im Dokument EP 0156512 die Herstellung einer verdünnten Lösung von Magnesium bis(2-ethylhexoxid) in Dodecan unter Verwendung von Iod beschrieben. Bei einer Reaktionstemperatur von 145 °C wird eine zehnstündige Reaktionszeit benötigt und das Produkt in Form einer viskosen Lösung erhalten. Um die extrem langen Reaktionszeiten zu umgehen, werden Magnesiumalkoholatlösungen deshalb im allgemeinen ausgehend von kommerziell erhältlichen Dialkylmagnesiumverbindungen (R₂Mg) hergestellt. Diese Syntheseroute hat jedoch den Nachteil, dass eine relativ teure Magnesiumquelle (nämlich die R₂Mg-Verbindungen, für deren Herstellung Halogenalkane benötigt werden), verwendet wird. Weiterhin impliziert sie eine Festlegung auf bestimmte Lösungsmittel, nämlich gesättigte Kohlenwasserstoffe: Dialkylmagnesiumverbindungen, beispielsweise Dibutylmagnesium, Butylethylmagnesium und Butyloctylmagnesium sind nur in gesättigten Kohlenwasserstoffen wie Hexan oder Heptan kommerziell erhältlich. Außerdem entstehen bei der Alkoholyse gemäß

R³R⁴Mg + 2 ROH → Mg(OR)₂ + R³H + R⁴H

unvermeidbar gesättigte Kohlenwasserstoffe (R³H und R⁴H, beispielsweise Butan oder Octan). Eine direkte Herstellung von Magnesiumalkoholaten in rein-aromatischen Lösungsmitteln wie Toluol oder Ethylbenzol ist deshalb ausgehend von kommerziell erhältlichen Dialkylmagnesiumverbindungen nicht möglich.

Eine weitere Synthesevariante zur Herstellung löslicher Erdalkalialkoholate besteht in der Umalkoholisierung unlöslicher Erdalkali-Alkoholate, hergestellt aus leicht flüchtigen Alkoholen (beispielsweise Ethanol) mit einem höher siedenden Alkohol, z.B.:

Mg(OR⁵)₂ + 2 ROH → Mg(OR)₂ + 2 R⁵OH

Nachteilig ist der relativ hohe, kostenintensive Aufwand dieser Methode, da das Alkoholat Mg(OR⁵)₂ zunächst aus dem flüchtigem Alkohol R⁵OH und Magnesiummetall hergestellt und isoliert werden muss, dann mit einem weniger flüchtigen Alkohol, beispielsweise 2-Ethylhexanol, umgesetzt werden und dann der flüchtigere Alkohol R⁵OH z.B. destillativ entfernt werden muss.

Die relativ hohe Viskosität von Magnesiumalkoxidlösungen wird durch Assoziationsphänomene verursacht. Aus dem Dokument US 6,734,134 ist bekannt, dass die Viskosität durch Zugabe von Alkylaluminiumverbindungen reduziert werden kann. Das bevorzugte Verhältnis zwischen Alkylaluminiumverbindung und Mg-Alkoholat liegt zwischen 0,001:1 und 1:1, mehr bevorzugt 0,01 bis 0,1:1 und ganz besonders bevorzugt 0,03 bis 0,05:1.

Aus der Schrift WO2010/146122 schließlich ist bekannt, gemischte Erdalkalialkoxidverbindungen M(OCH₂R⁶)₂₋ₓ(OR⁷)ₓ in Mischung mit einer Aluminiumverbindung Al(OCH₂R⁶)_{3-y}(OR⁷)_{y} in aprotischen Lösungsmitteln ausgehend von einem Erdalkalimetall und zwei verschiedenen Alkoholen herzustellen. Dabei ist
M ein Erdalkalimetall ausgewählt aus Mg, Ca, Ba, Sr;
OCH₂R⁶ ein Alkoxidrest bestehend aus mindestens 3 und höchstens 40 C-Atomen mit einer Verzweigung in 2-Position bezogen auf die O-Funktion, also R⁶ = -CHR⁸R⁹ mit R⁸, R⁹ = unabhängig voneinander Alkylreste C₁ - C₁₈;
R⁷ ein Alkylrest mit 2-15 C-Atomen, der entweder linear ist oder eine Verzweigung ≥ der 3-Position (bezogen auf die O-Funktion) aufweist
und die Summe von x und y eine Zahl zwischen 0,01 und 0,8, bevorzugt 0,02 und 0,3 und besonders bevorzugt 0,03 und 0,2.

Die mit Hilfe dieses Verfahrens hergestellten Produktlösungen weisen zwar relativ hohe Konzentrationen an Erdalkalialkoxidverbindungen auf (d.h. c_{Mg} > 0,5 mol/kg), jedoch sind die Viskositäten im Falle von Produkten mit relativ geringer Konzentration an viskositätsmindernden Al-Verbindungen (≤ 3 mol%, bezogen auf die gelöste Erdalkalimetallkonzentration) mit typisch ≥ 1.000 mPa.s (≥ 1.000 cP) bei Raumtemperatur (RT) noch unbefriedigend hoch. Für die Verwendung als Ziegler-Natta-Katalysatorträgermaterial ist gemäß dem Dokument US 6,734,134 eine geringe Al-Konzentration entscheidend.

Ferner können Dialkylmagnesiumverbindungen wie Butylethylmagnesium oder Dibutylmagnesium direkt zur Herstellung von Ziegler-Natta -Katalysatorträgermaterialien (chemisch gesehen MgCl₂) eingesetzt werden. Nachteilig am Einsatz von Dialkylmagnesiumverbindungen sind aber deren relativ hohe Herstellkosten sowie die Tatsache, dass Kohlenwasserstofflösungen solcher Metallorganyle pyrophor sind.

Diese pyrophoren Eigenschaften bedingen die Einhaltung spezieller und nachteiliger Transport-, Lager- und Handhabungsvorschriften.

Die Schrift US 3 903 019 A offenbart eine Zusammensetzung, die einen Komplex von Ba(t-BuO)₂/(Bu)₂Mg umfasst, der in Kohlenwasser-stoffen löslich ist und unter Verwendung von Ba-tert.alkoxid, Ba(O-CR₃)₂, worin R jeweils unabhängig aus der Gruppe bestehend aus Methyl, Ethyl und Propyl ausgewählt sind, und Dibutylmagnesium erhalten worden ist.

Die Schrift US 4 133 824 A offenbart einen kohlenwasserstofflöslichen organo-Magnesiumkomplex der Formel (R₂'Mg)ₘ.[(RO)₂Mg]ₙ, worin R eine Kohlenwasserstoffgruppe ist, R' eine primäre Alkylgruppe mit 1 bis 25 Kohlenstoffatomen ist, solche m und n Zahlen sind, das das Verhältnis von m/n 1 oder größer ist. Das offenbarte Molverhältnis der Erdalkalialkoxidverbindung zu der Erdalkalimetallalkylverbindung fordert einen Überschuss an der verwendeten Erdalkalimetallalkylkomponente.

Ebenfalls sind Mischungen aus Dialkoxymagnesium- und Dialkylmagnesium-Verbindungen bekannt. Die US 4 634 786 A offenbart ein Verfahren zur Herstellung von Kohlenwasserstofflösungen von Magnesiumdialkoxiden, wobei eine Magnesiummetall-Suspension oder eine Lösung einer Dialkyl-magnesiumverbindung in einem flüchtigen Kohlenwasserstoff mit Alkohol umgesetzt werden und der sich bei der Reaktion entwickelnde Wasserstoff entfernt wird. Die dabei im stöchiometrischen Überschuss eingesetzten Alkohole dienen für die eingesetzten Magnsiumdialkyle als Reaktionspartner zur Bildung von entsprechenden Magnesiumdialkoxide und/oder für die eingesetzten oder gebildeten Magnesiumdialkoxide als zusätzliches Lösungsmittel. So wird in Beispiel XVIII der US 4,634,786 die Herstellung einer Heptan-Cyclohexanlösung enthaltend einen 1:1-Komplex bestehend aus Magnesium 2-methyl-1-pentoxid und Dibutylmagnesium beschrieben. In den anderen Beispielen des genannten Patents werden jedoch exakt stöchiometrische Mengen an Dialkylmagnesiumverbindung und Alkoholen eingesetzt, so dass exakt stöchiometrisch zusammengesetzte Mg(OR¹R²)-Verbindungen (also solche, die frei sind von überschüssigem R₂Mg) gebildet werden. Den Beispielen I, II, III kann man Hinweise darauf entnehmen, dass es hinsichtlich der Viskosität günstig ist, einen Alkoholüberschuß einzusetzen. So wird in Beispiel 1 durch Zugabe von etwa 5 mol% 2-Methyl-1-pentanol die Viskosität der stöchiometrisch zusammengesetzten Produktlösung "spürbar" reduziert.

Ferner offenbart die Schrift EP 0 027 577 A1 ein Verfahren zur Herstellung von organo-Magnesiumalkoxiden R¹ₐR²_{b}Mg(OR³)_{c}(OR⁴)_{d}, worin R¹ bis R⁴ die gleichen oder verschiedene Alkylgruppen sind und R¹ und R² zusätzlich eine Arylgruppe oder eine Alkarylgruppe sein können, und a+b und c+d gleich 1 sind. Das Verfahren umfasst bei 50 bis 160°C die Umsetzung einer kohlenwasserstoff-löslichen Dialkylmagnesiumverbindung (R¹ₐR²_{b})₂Mg mit einer Verbindung Mg[(OR³)_{c}(OR⁴)_{d}]₂. R¹ kann beispielsweise Butyl sein und R² kann beispielsweise Octyl sein. R³ kann beispielsweise Ethyl sein und R⁴ kann beispielsweise Isopropyl sein, oder beide können beispielsweise Ethyl sein.

Die Erfindung hat sich die Aufgabe gestellt konzentrierte Erdalkalimetallalkoxidverbindungen in aprotischen Lösungsmitteln, insbesondere Kohlenwasserstoffen gesucht, die eine niedrige Al-Konzentration (z.B. < 5 mol%, bezogen auf Mg-Gehalt) aufweisen, die gleichzeitig eine niedrige Viskosität (z.B. < 500 mPa.s (< 500 cP) bei RT) aufweisen und die nicht pyrophor sind sowie Verfahren zu deren Herstellung anzugeben.

Die Aufgabe wird dadurch gelöst, dass Lösungen von Erdalkalialkoxidverbindungen M(OCH₂R⁶)_{2-a-b}(OR⁷)ₐ[O(CHR⁸)ₙOR⁹]_{b} in Mischung mit einer Erdalkalimetallalkylverbindung M(R¹⁰R¹¹) mit Erdalkalimetallkonzentration im Bereich von 0,2 bis 1,8 mmol/g in aprotischen Lösungsmitteln bereitgestellt werden, wobei
- M ein Erdalkalimetall ausgewählt aus Mg, Ca, Ba, Sr ist;
- OCH₂R⁶ ein Alkoxidrest bestehend aus mindestens 3 und höchstens 40 C-Atomen mit einer Verzweigung in 2-Position bezogen auf die O-Funktion ist, also R⁶ = -CHR¹²R¹³ mit R¹², R¹³ = unabhängig voneinander Alkylreste C₁ - C18;
- R⁷ ein Alkylrest mit 2-15 C-Atomen ist, der entweder linear ist oder eine Verzweigung ≥ der 3-Position (bezogen auf die O-Funktion) aufweist
- R⁸ ein Alkylrest mit 1-6 C-Atomen ist, der entweder linear ist oder eine Verzweigung ≥ der 3-Position (bezogen auf die O-Funktion) aufweist
- R⁹ ein Alkylrest mit 2-15 C-Atomen ist, der entweder linear ist oder eine Verzweigung aufweist
- R¹⁰ und R¹¹ beliebige Alkylreste mit 1-15 C-Atomen sind,
- n = eine ganze Zahl von 1 bis 4 ist,
- a + b ≤ 2, wobei a und b Werte von 0 bis 2 annehmen können und
- das Molverhältnis M(OCH₂R⁶)_{2-a-b}(OR⁷)ₐ[O(CHR⁸)ₙOR⁹]_{b} zu M(R¹⁰R¹¹) im Bereich von 99:1 bis 70:30 und bevorzugt von 95:5 bis 80:20 liegt.

Es wurde überraschend beobachtet, dass durch Zusatz bereits kleiner Mengen von Erdalkalimetallalkylen zu Lösungen von Erdalkalialkoxidverbindungen in aprotischen Lösungsmitteln deren Viskosität drastisch vermindert werden kann. Dies steht im Gegensatz zu der aus US 4,634,786 bekannten technischen Lehre, dass nämlich nur die Zugabe weiteren Alkohols zu stöchiometrisch zusammengesetzten Magnesiumalkoxidlösungen viskositätsmindernd wirkt.

Die erfindungsgemäßen Lösungen enthalten von 0,1 bis 30 mol%, bevorzugt 1 bis 20 mol% und besonders bevorzugt 3-15 mol% Aktivbase R₂Mg, bestimmt durch Direkttitration mit sec-Butanol und Bichinolin als Indikator und bezogen auf insgesamt in Lösung befindliches Erdalkalimetall M.

Vorzugsweise ist in der erfindungsgemäßen Lösungen weiterhin eine Aluminiumverbindung Al(OCH₂R⁶)_{3-c-d}(OR⁷)_{c}[O(CHR⁸)ₙOR⁹]_{d} enthalten, wobei
- OCH₂R⁶ ein Alkoxidrest bestehend aus mindestens 3 und höchstens 40 C-Atomen mit einer Verzweigung in 2-Position bezogen auf die O-Funktion ist, also R⁶ = -CHR¹²R¹³ mit R¹², R¹³ = unabhängig voneinander Alkylreste C₁ - C₁₈;
- R⁷ ein Alkylrest mit 2-15 C-Atomen ist, der entweder linear ist oder eine Verzweigung ≥ der 3-Position (bezogen auf die O-Funktion) aufweist;
- R⁸ ein Alkylrest mit 1-6 C-Atomen ist, der entweder linear ist oder eine Verzweigung ≥ der 3-Position (bezogen auf die O-Funktion) aufweist;
- R⁹ ein Alkylrest mit 2-15 C-Atomen ist, der entweder linear ist oder eine Verzweigung aufweist,
- n = eine ganze Zahl von 1 bis 4 ist, wobei
c + d ≤ 3 ist und c sowie d Werte von 0 bis 3 annehmen können und wobei der Gehalt an Aluminiumverbindung Al(OCH₂R⁶)_{3-c-d}(OR⁷)_{c}[O(CHR⁸)ₙOR⁹]_{d} bezogen auf das gelöste Erdalkalimetall im Bereich von 0 bis etwa 20 mol %, bevorzugt von 0,2 bis 5 mol%, besonders bevorzugt von 0,5 bis 4 mol% liegt.

Das aprotische Lösungsmittel ist oder enthält entweder einen oder mehrere aliphatische Verbindungen mit 5 bis 20 C-Atomen, wobei sowohl zyklische als auch offenkettige Verbindungen möglich sind. Bevorzugt sind: Cyclohexan, Methylcyclohexan, Hexan, Heptan, Octan, Nonan, Dekan, Dodecan, Dekalin sowie handelsübliche Siedeschnitte (Benzinfraktionen).

Das aprotische Lösungsmittel kann weiterhin Aromaten enthalten oder daraus bestehen. Bevorzugt sind: Benzol, Toluol, Ethylbenzol, Xylole sowie Cumol.

In einer weiteren Ausführungsform der Erfindung kann die erfindungsgemäße Erdalkalialkoxidlösung noch polare, aprotische Lösungsmittel wie z.B. Ether oder tertiäre Amine enthalten.

Der in 2-Position verzweigte Alkohol (HOCH₂R⁶) ist besonders bevorzugt ausgewählt aus der Gruppe bestehend aus: Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder einer beliebigen Mischung aus mindestens zwei der aufgeführten Alkohole. Der primäre Alkohol (HOR⁷) ist bevorzugt ausgewählt aus der Gruppe bestehend aus: Ethanol, Propanol, Butanol, Pentanol, Hexanol, Octanol, Decanol, Dodekanol, 3-Methylbutan-1-ol oder einer beliebigen Mischung aus mindestens zwei der aufgeführten Alkohole. Der eine Alkoxyfunktion enthaltende Alkohol HO(CHR⁸)ₙOR⁹ ist bevorzugt ein C₂-C₄ - Glykolmonoether, beispielsweise 2-Ethoxyethanol, 3-Ethoxy-1-propanol, 3-Ethoxy-1-butanol, 2-(2-Ethylhexoxy)ethanol, 2-Butoxyethanol, 2-Hexyloxyethanol sowie 1,3-Propylenglycolmonobutylether oder eine beliebige Mischung aus mindestens zwei der aufgeführten Alkohole.

Die erfindungsgemäßen Produkte können beispielsweise nach zwei unterschiedlichen Verfahren hergestellt werden.

Das erste Verfahren zur Herstellung von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹), die beide wie in Anspruch 1 definiert sind, ist dadurch gekennzeichnet, dass ein Erdalkalimetall oder eine Erdalkalimetallmischung in einem aprotischen Lösungsmittel mit den Alkoholen umgesetzt wird und nach Beendigung dieser Reaktion die Lösung einer Erdalkalimetallalkylverbindung M(R10R11) zugegeben wird, wobei das Molverhältnis der Erdalkali-oxidverbindung zur Erdalkalimetallalkylverbindung im Bereich von 99:1 bis 70:30, besonders bevorzugt von 95:5 bis 80:20 liegt.

Das erste Verfahren geht von handelsüblichem Erdalkalimetall, bevorzugt Magnesiummetall, dieses bevorzugt in Pulver-, Granulat- oder Spanform, aus. Das Metall wird in einem wasserfreien aprotischen Lösungsmittel, bevorzugt aromatischen oder aliphatischen Kohlenwasserstoffen in einem inertisierten, d.h. getrockneten und mit Schutzgas (Stickstoff oder Argon) versehenem Rührwerksbehälter vorgelegt. Dann kann eine Alkylaluminiumverbindung, z.B. Trialkylaluminium wie Triethylaluminium, Tributylaluminium, ein Alkylaluminiumhydrid wie Dibutylaluminiumhydrid, ein Alkylaluminiumhalogenid wie Dibutylaluminiumchlorid oder eine Alkoxyaluminiumverbindung wie beispielsweise Diethylaluminiumethoxid zugegeben werden. Im Allgemeinen liegt das Molverhältnis Alkylaluminiumverbindung zu den Alkoholen von 0 bis 0,1 zu 1, bevorzugt von 0,005 bis 0,04 zu 1. Die Aluminiumverbindung kann auch ganz oder teilweise nach dem oder zu den Alkohol(en) zugegeben werden.

Dann werden die gewünschten Alkohole, d.h. der eine Alkoxyfunktion enthaltende Alkohol HO(CHR⁸)ₙOR⁹ und/oder ein verzweigter Alkohol HOCH₂R⁶ und/oder ein unverzweigter oder eine Verzweigung ≥ der 3-Position aufweisender primärer Alkohol mit 2-15 C-Atomen (HOR⁷) entweder als Mischung oder hintereinander zugegeben. Bevorzugt wird zunächst der primäre Alkohol R⁷OH zugegeben, dann erst die Alkohole ausgewählt aus den anderen Stoffgruppen. Die Zugabe kann bei Temperaturen zwischen von 0 und 180 °C erfolgen, bevorzugt zwischen etwa 40 und 140 °C. Ganz besonders bevorzugt erfolgt sie am Siedepunkt des verwendeten Lösungsmittels, also z.B. im Falle von Toluol bei etwa 110 °C. Die Reaktionszeit richtet sich nach der Reaktivität des verwendeten Erdalkalimetalls, insbesondere des Magnesiums, sowie der Acidität des verwendeten Alkohols, dem stöchiometrischen Verhältnis zwischen Erdalkalimetall, insbesondere Magnesium, und den Alkoholen sowie der Reaktionstemperatur. Wird das Erdalkalimetall, insbesondere das Magnesium, im Überschuss eingesetzt (bevorzugt von 1 bis 300 mol%, besonders bevorzugt von 10 bis 100 mol%), reichen bei der Rückflussfahrweise 1 bis 6 Stunden Reaktionszeit aus. Es wird bevorzugt so lange reagiert, bis praktisch aller Alkohol umgesetzt ist, d.h. dessen Konzentration < 0,01 mmol/g, bevorzugt < 0,001 mmol/g liegt.

Nach Beendigung der Reaktion, erkennbar am Versiegen des Wasserstoffstromes, wird zu der relativ viskosen Reaktionsmischung die Lösung einer Erdalkalimetallalkylverbindung, z.B. MgR¹⁰R¹¹, zugegeben. Es wird in der Regel ein weiterer kurzfristiger Viskositätsanstieg beobachtet, der sich jedoch nach Zugabe von wenigen mol% MgR¹⁰R¹¹ ins Gegenteil verkehrt, d.h. die Reaktionslösung wird überraschenderweise fast schlagartig niedrigviskos. In Abhängigkeit von den verwendeten Alkoholen und anderen Parametern tritt dieser Effekt nach Zugabe von 1 - 20 mol% Metallalkylverbindung auf. Die weitere Zugabe von MgR¹⁰R¹¹ bewirkt hingegen nur noch einen handhabungstechnisch unbedeutenden weiteren Viskositätsabfall. Wird beispielsweise eine etwa 30 Gew.-%ige Heptanlösung von Magnesium-bis(2-ethylhexoxid) enthaltend knapp 3 mol% Al mit 9 oder 4 mol% einer Dialkylmagnesiumverbindung, beispielsweise Butylethylmagnesium umgesetzt, so sinken die Viskositäten von ≥ 1000 mPa.s (≥ 1000 cP) auf ca. 50 bzw. 100 mPa.s (50 bzw. 100 cP) bei RT.

Das zweite Verfahren zur Herstellung von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹), die beide wie in Anspruch 1 definiert sind, ist dadurch gekennzeichnet, dass die Lösung einer Metallalkylverbindung M(R¹⁰R¹¹) in einem aprotischen Lösungsmittel mit den Alkoholen HOCH₂R⁶, HOR⁷ und/oder HO(CHR⁸)ₙOR⁹ versetzt werden, wobei das halbe Molverhältnis der Mol-Summe aller Alkohole zur Erdalkalimetallalkylverbindung im Bereich von 99:1 bis 70:30, besonders bevorzugt von 95:5 bis 80:20, liegt.

Das zweite bevorzugte Herstellverfahren geht von Lösungen von Dialkylmagnesiumverbindungen in aprotischen Lösungsmitteln aus. Zu diesen wird der oder werden die gewünschte(n) Alkohol(e) in einer Stöchiometrie zugegeben, die zu den erfindungsgemäßen Produkten führt. Die Reihenfolge der Zugabe ist beliebig; es kann auch eine vorgefertigte Alkoholmischung verwendet werden. Weiterhin ist es möglich, den Alkohol oder die Alkohole, bevorzugt in Mischung mit einem aprotischen Lösungsmittel vorzulegen und die Dialkylmagnesiumkomponente zuzugeben. Schließlich ist auch eine Simultandosierung in vorgelegtes Lösungsmittel denkbar.

Die nach erfindungsgemäßen Verfahren hergestellten Produkte sind überraschenderweise trotz hoher Erdalkalimetall-Konzentration von ≥ 0,5 mol/kg, bevorzugt ≥ 1,0 mol/kg sehr wenig viskos. Die Erdalkalimetall - Konzentrationen liegen bevorzugt im Bereich von ca. 0,4 bis 1,6 mmol/g, besonders bevorzugt von 0,7 bis 1,4 mmol/g. Die bei Raumtemperatur gemessenen Viskositäten liegen bei Mg-Konzentrationen ≥ 1 mmol/g und ≤ 1,5 mmol/g im allgemeinen unter 300 mPa.s (300 cP), bevorzugt unter 200 mPa.s (200 cP), besonders bevorzugt unter 100 mPa.s (100 cP).

Der Gehalt an gelöstem Aluminium liegt bezogen auf gelöstes Erdalkalimetall im Bereich von 0 bis ca. 20 mol %, bevorzugt im Bereich von 0,2 bis 15 mol %, besonders bevorzugt im Bereich von 0,5 bis 4 mol%.

Die erfindungsgemäßen Produkte werden für die Herstellung von Polymerisationskatalysatoren, insbesondere heterogenisierte Polyolefinkatalysatoren vom Ziegler-Natta-Typ verwendet. Ferner können Sie in der organischen Synthese, beispielsweise als Basen eingesetzt werden.

### Beispiele

Alle Umsetzungen wurden in trockenen, mit Argon inertisierten Glasapparaturen vorgenommen. Es wurden handelsübliche Magnesiumspäne eingesetzt. Die Konzentrationen an Mg und Al wurden mittels ICP (induktiv gekoppeltes Plasma) gemessen.

Die Aktivbase wird mittels Direkttitration mit 1M 2-Butanollösung in Hexan gegen 2,2-Bichinolin als Indikator ermittelt. Umschlag von rot nach grau.

### Beispiel 1: Herstellung einer 35%igen Lösung von Magnesium bis(2-ethylhexoxid) im Gemisch mit 6 mol% Dibutylmagnesium in Heptan

In einem 0,5 L-Doppelmantelglasreaktor mit Rückflusskühler und Tropftrichter wurden 32,0 g Magnesiumspäne und 352 g Heptan vorgelegt. Dann wurden 11,3 g einer 20 Gew.-%igen Lösung von Triethylaluminium in Heptan, 1,8 g Ethanol sowie 171,9 g 2-Ethylhexanol zugegeben und zum Siedepunkt aufgeheizt. Es wurde 4 Stunden refluxiert, wobei sich 14,6 I Gas entwickelten und eine zähe Lösung von Magnesium bis(2-ethylhexoxid) entstand. Es wurde eine Probe entnommen und deren Viskosität ermittelt (1025 mPa.s (1025 cP) bei 25°C)).

Das Reaktionsgemisch wurde auf ca. 80 °C abgekühlt und 54,6 g einer Dibutylmagnesiumlösung in Hexan (Mg = 1,08 mmol/g) zugegeben. Nach der Zugabe entstand eine dünnflüssige, leicht handhabbare Lösung. Die hellgraue Suspension wurde abgehebert und filtriert. Es wurden 579 g einer nichtviskosen Flüssigkeit mit einem Magnesiumgehalt von 1,22 mmol/g erhalten. Die Produktlösung enthielt weiterhin 0,030 mmol/g Aluminium und sie wies einen Aktivbasegehalt von 0,15 mmol/g (entspricht 0,075 mmol/g Bu₂Mg, ca. 6 mol%) auf.
Ausbeute: 98 % d. Th.
Viskosität (Brookfield, 25°C): 33 mPa.s (33 cP)
Im UN-Test N.2, N.3 erwies sich die Produktlösung als nicht pyrophor.

### Beispiel 2: Herstellung einer 29 %igen Magnesiumdecanolatlösung in Hexan im Gemisch mit 14 mol% Dibutylmagnesium

In einem 0,5 L-Doppelmantelglasreaktor mit Rückflusskühler und Tropftrichter wurden 82,0 g einer Dibutylmagnesiumlösung in Hexan (Mg = 1,11 mmol/g, 91 mmol) vorgelegt. Dann wurden 23,6 g n-Decanol (149 mmol) unter intensivem Rühren zugegeben. An der Eintropfstelle entstand vorübergehend ein gelartiges Reaktionsprodukt, welches sich beim Rühren aber vollständig löste. Nach Dosierende war eine nicht-viskose farblose und klare Lösung entstanden.
Ausbeute: 104 g Lösung
Gesamt-Magnesiumgehalt: 0,88 mmol/g
Aktivbasegehalt: 0,24 mmol/g
Viskosität (Brookfield, 25°C): 4,8 mPa.s (4,8 cP)
Im UN-Test N.2, N.3 erwies sich die Produktlösung als nicht pyrophor.

### Vergleichsbeispiel 1: Herstellung einer etwa 30 % Magnesiumdecanolatlösung in Hexan

In einem 0,5 L-Doppelmantelglasreaktor mit Rückflusskühler und Tropftrichter wurden 85,0 g einer Dibutylmagnesiumlösung in Hexan (Mg = 1,11 mmol/g, 94 mmol) vorgelegt. Dann wurden 31,2 g n-Decanol (197 mmol) unter intensivem Rühren zugegeben. Nach Zugabe von etwa 90 % der Gesamtalkoholmenge löste sich die an der Eintropfstelle entstehende gelartige Phase immer langsamer und dann gar nicht mehr auf. Nach Dosierende war ein stichfestes Gel entstanden, das auch bei Erwärmen (ca. 80°C) nicht verflüssigt werden konnte.

Wegen der gelartigen Konsistenz konnte mittels Spritze keine Probe entnommen werden.

### Beispiel 3: Herstellung einer 35%igen Lösung von Magnesium bis(2-ethylhexoxid)/ Magnesiumdecanolat (75:25) im Gemisch mit etwa 5 mol% Butylethylmagnesium in Heptan

In einem 0,5 L-Doppelmantelglasreaktor mit Rückflusskühler und Tropftrichter wurden 32,0 g Magnesiumspäne und 352 g Heptan vorgelegt. Dann wurden 11,3 g einer 20 Gew.-%igen Lösung von Triethylaluminium in Heptan, 1,8 g Ethanol sowie ein Gemisch aus 128,9 g 2-Ethylhexanol und 52,2 g 1-Decanol zugegeben und zum Siedepunkt aufgeheizt. Es wurde 3,5 Stunden refluxiert, wobei sich 16,0 I Gas entwickelten und eine zähe Lösung des gemischten Magnesiumalkoxids entstand. Es wurde eine Probe entnommen und deren Viskosität ermittelt (3800 mPa.s (3800cP) bei 25°C).

Es wurde auf 100 °C abgekühlt und 55,1 g einer Butylethylmagnesiumlösung in Heptan (Mg = 1,09 mmol/g) zugegeben. Nach der Zugabe war eine dünnflüssige, leicht handhabbare Lösung entstanden. Die hellgraue Suspension wurde abgehebert und filtriert. Es wurden 534 g einer nichtviskosen Flüssigkeit mit einem Magnesiumgehalt von 1,19 mmol/g isoliert. Die Produktlösung enthielt weiterhin 0,033 mmol/g Aluminium und sie wies einen Aktivbasegehalt von 0,11 mmol/g (entspr. 0,055 mmol/g BuMgEt, 4,6 mol%) auf.
Ausbeute: 88 % d. Th.
Viskosität (Brookfield, 25°C): 16 mPa.s (16 cP)
Im UN-Test N.2, N.3 erwies sich die Produktlösung als nicht pyrophor.

### Beispiel 4: Herstellung einer 34%igen Lösung von Magnesium bis(2-ethylhexoxid) im Gemisch mit 5 mol% Butylethylmagnesium in Toluol

In einem 0,5 L-Doppelmantelglasreaktor mit Rückflusskühler und Tropftrichter wurden 32,0 g Magnesiumspäne und 352 g Toluol vorgelegt. Dann wurden 9,0 g einer 25% Gew.-%igen Lösung von Triethylaluminium in Toluol, 1,8 g Ethanol sowie 171,9 g 2-Ethylhexanol zugegeben und zum Siedepunkt aufgeheizt. Es wurde knapp 4 Stunden refluxiert, wobei sich 16,4 l Gas entwickelten und eine zähe Lösung des Magnesiumalkoxids entstand.

Dann wurde auf 100 °C abgekühlt und 56,5 g einer Dibutylmagnesiumlösung in Heptan (Mg = 1,08 mmol/g) zugegeben. Nach der Zugabe war eine dünnflüssige, leicht handhabbare Lösung entstanden. Die hellgraue Suspension wurde abgehebert und filtriert. Es wurden 576 g einer nichtviskosen Flüssigkeit mit einem Magnesiumgehalt von 1,21 mmol/g isoliert. Die Produktlösung enthielt weiterhin 0,030 mmol/g Aluminium und sie wies einen Aktivbasegehalt von 0,13 mmol/g (entspricht 0,065 mmol/g BuMgEt, 5,4 mol%) auf.
Ausbeute: 97 % d. Th.
Viskosität (Brookfield, 25°C): 94 mPa.s (94 cP)
Im UN-Test N.2, N.3 erwies sich die Produktlösung als nicht pyrophor.

Dem Vergleichsbeispiel 1 sowie einem Vergleich der Viskositätsdaten vor und nach der Zugabe von Dialkylmagnesiumlösung in den Beispielen 1 und 3 kann der positive Einfluß, herbeigeführt durch die Zugabe von Dialkylmagnesiumlösung zu Magnesiumalkoxidlösungen (Beispiele 1 und 3) bzw. die Verwendung eines Alkoholunterschusses bei der Umsetzung mit Dialkylmagnesiumlösung (Beispiel 2 und Vergleichsbeispiel 1), entnommen werden.

Während alle erfindungsgemäß hergestellten Produktlösungen mit Mg-Konzentrationen zwischen 0,88 und 1,22 mmol/g und Al-Konzentrationen von ≤ 3 mol% sehr gut handhabbar und dünnflüssig waren (Viskositäten bei 25°C < 100 mPa.s (< 100 cP)), waren die Dialkylmagnesium-freien Produktlösungen ausgesprochen viskos: die Viskositäten der Flüssigprodukte lagen zwischen > 1000 und 3800 mPa.s (> 1000 und 3800 cP). Wird kein in 2-Stellung verzweigter Alkohol (HOCH₂R⁶ bestehend aus mindestens 3 und höchstens 40 C-Atomen mit einer Verzweigung in 2-Position bezogen auf die O-Funktion, also R⁶ = -CHR¹²R¹³ mit R¹², R¹³ = unabhängig voneinander Alkylreste C₁ - C₁₈) eingesetzt, sondern lediglich unverzweigte Alkohole, so entsteht bei vollständiger Umsetzung bzw. Verwendung eines leichten Alkoholüberschusses ein nicht pump- oder sonstwie transferierbares gelartiges Produkt (Vergleichsbeispiel 1). Dagegen wird bei Anwesenheit von etwa 14 mol% Dibutylmagnesium ein wasserartig dünnflüssiges Produkt erhalten. Ein solches Produkt ist nach US 4,634,786 nicht herstellbar, da nach diesem Dokument zum Stand der Technik "(a) aliphatische 2-alkyl-substituierte primäre Monoalkohole; oder (b) Mischungen aus genannten (a) Alkoholen mit C₃ - C₁₂ aliphatischen sekundären oder tertiären Alkoholen; oder (c) Mischungen aus genannten (a) Alkoholen mit C₁ - C₁₂ aliphatischen primären linearen unsubstituierten Alkoholen; wobei die Molverhältnisse genannter (a) zu genannter (b), und genannter (a) zu genannter (c) Alkohole 1 für genannte (a) Alkohole zu 0,1 bis 2 von genannten (b) und genannten (c) Alkoholen (beträgt)", also in jedem Falle in 2-Stellung verzweigte (a) Alkohole, benötigt werden.

Alle erfindungsgemäßen Produktlösungen sind nicht pyrophor.

## Patentansprüche

1. Lösungen von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹) mit Erdalkalimetallkonzentration im Bereich von 0,2 bis 1,8 mmol/g in aprotischen Lösungsmitteln, wobei
• M ein Erdalkalimetall ausgewählt aus Mg, Ca, Ba, Sr ist;
• OCH₂R⁶ ein Alkoxidrest bestehend aus mindestens 3 und höchstens 40 C-Atomen mit einer Verzweigung in 2-Position bezogen auf die O-Funktion ist, also R⁶ = -CHR¹²R¹³ mit R¹², R¹³ = unabhängig voneinander Alkylreste C₁-C₁₈;
• R⁷ ein Alkylrest mit 2-15 C-Atomen ist, der entweder linear ist oder eine Verzweigung ≥ der 3-Position (bezogen auf die O-Funktion) aufweist;
• R⁸ ein Alkylrest mit 1-6 C-Atomen ist, der entweder linear ist oder eine Verzweigung ≥ der 3-Position (bezogen auf die O-Funktion) aufweist;
• R⁹ ein Alkylrest mit 2-15 C-Atomen ist, der entweder linear ist oder eine Verzweigung aufweist;
• R¹⁰ und R¹¹ beliebige Alkylreste mit 1-15 C-Atomen sind,
• n = eine ganze Zahl von 1 bis 4 ist,
• a + b ≤ 2, a und b Werte von 0 bis 2 annehmen können
und
• das Molverhältnis M(OCH₂R⁶)_{2-a-b}(OR⁷)ₐ[0(CHR⁸)ₙOR⁹]_{b} zu M(R¹⁰R¹¹) im Bereich von 99:1 bis 70:30 liegt.

2. Lösungen nach Anspruch 1, **dadurch gekennzeichnet, dass** die Erdalkalimetallkonzentration bevorzugt im Bereich von 0,4 bis 1,6 mmol/g, besonders bevorzugt von 0,7 bis 1,4 mmol/g liegt.

3. Lösungen nach Anspruch 1 oder 2, **dadurch gekennzeichnet, dass** die Raumtemperatur-Viskositäten bei Mg-Konzentrationen von ≥ 1 mmol/g bis ≤ 1,6 mmol/g ≤ 300 cP, bevorzugt ≤ 200 cP, besonders bevorzugt ≤ 100 cP liegen.

4. Lösungen nach Anspruch 1 bis 3, **dadurch gekennzeichnet, dass** das Molverhältnis M(OCH₂R⁶)_{2-a-b}(OR⁷)ₐ[0(CHR⁸)ₙOR⁹]_{b} zu M(R¹⁰R¹¹)im Bereich von 95:5 bis 80:20 liegt.

5. Lösungen nach Anspruch 1 bis 4, **dadurch gekennzeichnet, dass** eine Aluminiumverbindung Al(OCH₂R⁶)_{3-c-d}(OR⁷)_{c}[O(CHR⁸)ₙ OR⁹]_{d} enthalten ist, wobei
• OCH₂R⁶ ein Alkoxidrest bestehend aus mindestens 3 und höchstens 40 C-Atomen mit einer Verzweigung in 2-Position bezogen auf die O-Funktion ist, also R⁶ = -CHR¹²R¹³ mit R¹², R¹³ = unabhängig voneinander Alkylreste C₁-C₁₈;
• R⁷ ein Alkylrest mit 2-15 C-Atomen ist, der entweder linear ist oder eine Verzweigung ≥ der 3-Position (bezogen auf die O-Funktion) aufweist;
• R⁸ ein Alkylrest mit 1-6 C-Atomen ist, der entweder linear ist oder eine Verzweigung ≥ der 3-Position (bezogen auf die O-Funktion) aufweist;
• R⁹ ein Alkylrest mit 2-15 C-Atomen ist, der entweder linear ist oder eine Verzweigung aufweist;
• n = eine ganze Zahl von 1 bis 4 ist,
• c + d ≤ 3 ist und c sowie d Werte von 0 bis 3 annehmen können und wobei der Gehalt an Aluminiumverbindung Al(OCH₂R⁶)_{3-c-d}(OR⁷)_{c}[O(CHR⁸)ₙOR⁹]_{d} bezogen auf das gelöste Erdalkalimetall im Bereich von 0 bis etwa 20 mol%, bevorzugt von 0,2 und 15 mol%, besonders bevorzugt von 0,5 und 4 mol% liegt.

6. Lösungen nach Anspruch 1 bis 5, **dadurch gekennzeichnet, dass** als eine Alkoxyfunktion enthaltender Alkohol HO(CHR⁸)ₙOR⁹ bevorzugt ein C₂-C₄-Glykolmonoether, beispielsweise 2-Ethoxyethanol, 3-Ethoxy-1-propanol, 3-Ethoxy-1-butanol, 2-(2-Ethylhexoxy)ethanol, 2-Butoxyethanol, 2-Hexyloxyethanol sowie 1,3-Propylenglycolmonobutylether oder einer beliebigen Mischung aus mindestens zwei der aufgeführten Alkohole; als in 2-Stellung verzweigter Alkohol (HOCH₂R⁶) Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder eine beliebige Mischung aus mindestens zwei der aufgeführten Alkohole und als unverzweigter oder eine Verzweigung ≥ der 3-Position aufweisender primärer Alkohol mit 2-15 C-Atomen (HOR⁷) Ethanol, Propanol, Butanol, Pentanol, Hexanol, Octanol, Decanol, Dodekanol, 3-Methylbutan-1-ol oder eine beliebige Mischung aus mindestens zwei der aufgeführten Alkohole eingesetzt werden.

7. Lösungen nach Anspruch 1 bis 6, **dadurch gekennzeichnet, dass** sie von 0,1 bis 30 mol%, bevorzugt 1 bis 20 mol% und besonders bevorzugt 3-15 mol% Aktivbase R₂Mg, bestimmt durch Direkttitration mit sec-Butanol und Bichinolin als Indikator und bezogen auf insgesamt in Lösung befindliches Erdalkalimetall M enthalten und dass sie nach UN-Test nicht pyrophor sind.

8. Verfahren zur Herstellung von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹), beide wie in Anspruch 1 definiert, **dadurch gekennzeichnet, dass** ein Erdalkalimetall oder eine Erdalkalimetallmischung in einem aprotischen Lösungsmittel mit den Alkoholen umgesetzt wird und nach Beendigung dieser Reaktion die Lösung einer Erdalkalimetallalkylverbindung M(R¹⁰R¹¹) zugegeben wird, wobei das Molverhältnis der Erdalkali-oxidverbindung zur Erdalkalimetallalkylverbindung im Bereich von 99:1 bis 70:30, besonders bevorzugt von 95:5 bis 80:20 liegt.

9. Verfahren nach Anspruch 8, **dadurch gekennzeichnet, dass** eine oder mehrere Alkylaluminiumverbindungen ausgewählt aus Trialkyl-, Alkyl-Alkoxy- und/oder Alkyl-HalogenidVerbindung zugegeben wird und die Al-Konzentration bezogen auf das gelöste Erdalkalimetall im Bereich von 0 bis etwa 20 mol %, bevorzugt im Bereich von 0,2 bis 15 mol %, besonders bevorzugt von 0,5 bis 4 mol% liegt.

10. Verfahren nach Anspruch 8 oder 9, **dadurch gekennzeichnet, dass** als Lösemittel Kohlenwasserstoffe eingesetzt werden, wobei entweder aliphatische Lösemittel ausgewählt aus der Gruppe bestehend aus Cyclohexan, Methylcyclohexan, Hexan, Heptan, Octan, Nonan, Dekan, Dodecan, Dekalin sowie handelsübliche Siedeschnitte (Benzinfraktionen) oder aromatische Lösemittel ausgewählt aus der Gruppe bestehend aus Benzol, Toluol, Ethylbenzol, Xylolen sowie Cumol verwendet werden.

11. Verfahren nach Anspruch 8 bis 10, **dadurch gekennzeichnet, dass** die Umsetzung bei Temperaturen zwischen etwa 0 und 180°C erfolgt, bevorzugt zwischen etwa 40 und 140°C, insbesondere bei der Siedetemperatur des Lösemittels.

12. Verfahren nach Anspruch 8 bis 11, **dadurch gekennzeichnet, dass** als eine Alkoxyfunktion enthaltender Alkohol HO(CHR⁸)ₙOR⁹ bevorzugt ein C₂-C₄-Glykolmonoether, beispielsweise 2-Ethoxyethanol, 3-Ethoxy-1-propanol, 3-Ethoxy-1-butanol, 2-(2-Ethylhexoxy) ethanol, 2-Butoxyethanol, 2-Hexyloxyethanol sowie 1,3-Propylenglycolmonobutylether oder einer beliebigen Mischung aus mindestens zwei der aufgeführten Alkohole; als in 2-Stellung verzweigter Alkohol (HOCH₂R⁶) Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder eine beliebige Mischung aus mindestens zwei der aufgeführten Alkohole und als unverzweigter oder eine Verzweigung ≥ der 3-Position aufweisender primärer Alkohol mit 2-15 C-Atomen (HOR⁷) Ethanol, Propanol, Butanol, Pentanol, Hexanol, Octanol, Decanol, Dodekanol, 3-Methylbutan-1-ol oder eine beliebige Mischung aus mindestens zwei der aufgeführten Alkohole eingesetzt werden.

13. Verfahren zur Herstellung Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹), beide wie in Anspruch 1 definiert, **dadurch gekennzeichnet, dass** die Lösung einer Metallalkylverbindung M(R¹⁰R¹¹)in einem aprotischen Lösungsmittel mit den Alkoholen HOCH₂R⁶, HOR⁷ und/oder HO(CHR⁸)ₙOR⁹ versetzt werden, wobei das halbe Molverhältnis der Mol-Summe aller Alkohole zur Erdalkalimetallalkylverbindung im Bereich von 99:1 bis 70:30, besonders bevorzugt von 95:5 bis 80:20, liegt.

14. Verwendung von Lösungen von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹) gemäß Anspruch 1 zur Herstellung von Polymerisationskatalysatoren, insbesondere heterogenisierten Polyolefinkatalysatoren vom Ziegler-Natta-Typ.

15. Verwendung von Lösungen von Erdalkalialkoxidverbindungen M(OCH₂R⁶)_{2-a-b}(OR⁷)ₐ[0(CHR⁸)ₙOR⁹]_{b} in Mischung mit einer Erdalkalimetallalkylverbindung M(R¹⁰R¹¹)gemäß Anspruch 1 in der organischen Synthese, beispielsweise als Base.

## Claims

1. Solutions of alkaline earth alkoxide compounds
M(OCH₂R⁶)_{2-a-b}(OR⁷)ₐ[O(CHR⁸)ₙOR⁹]_{b} in a mixture with an alkaline earth metal alkyl compound M(R¹⁰R¹¹) with an alkaline earth metal concentration in the range from 0.2 to 1.8 mmol/g in aprotic solvents, wherein
• M is an alkaline earth metal selected from Mg, Ca, Ba, Sr;
• OCH₂R⁶ is an alkoxide radical consisting of at least 3 and at most 40 carbon atoms with a branch in the 2-position relative to the O function, i.e., R⁶ = -CHR¹²R¹³ with R¹², R¹³ = independently of one another alkyl radicals C₁-C₁₈;
• R⁷ is an alkyl radical with 2-15 C atoms, which is either linear or has a branch ≥ the 3-position (relative to the O function);
• R⁸ is an alkyl radical with 1-6 C atoms, which is either linear or has a branch ≥ the 3-position (relative to the O function);
• R⁹ is an alkyl radical with 2-15 C atoms, which is either linear or has a branch;
• R¹⁰ and R¹¹ are any alkyl radicals with 1-15 C atoms;
• n = an integer from 1 to 4;
• a + b ≤ 2, a and b can assume values from 0 to 2; and
• the molar ratio of M(OCH₂R⁶)_{2-a-b}(OR⁷)ₐ[O(CHR⁸)ₙOR⁹]_{b} to M(R¹⁰R¹¹) is in the range from 99:1 to 70:30.

2. The solutions according to claim 1, **characterized in that** the alkaline earth metal concentration is preferably in the range from 0.4 to 1.6 mmol/g, particularly preferred from 0.7 to 1.4 mmol/g.

3. The solutions according to claim 1 or 2, **characterized in that** the room temperature viscosities at Mg concentrations of ≥ 1 mmol/g to ≤ 1.6 mmol/g are ≤ 300 cP, preferably ≤ 200 cP, particularly preferred ≤ 100 cP.

4. The solutions according to claim 1 to 3, **characterized in that** the molar ratio of M(OCH₂R⁶)_{2-a-b}(OR⁷)ₐ[O(CHR⁸)ₙOR⁹]_{b} to M(R¹⁰R¹¹) is in the range from 95:5 to 80:20.

5. The solutions according to claim 1 to 4, **characterized in that** an aluminum compound Al(OCH₂R⁶)_{3-c-d}(OR⁷)_{c}[O(CHR⁸)ₙOR⁹]_{d} is present, wherein
• OCH₂R⁶ is an alkoxide radical consisting of at least 3 and at most 40 carbon atoms with a branch in the 2-position relative to the O function, i.e., R⁶ = -CHR¹²R¹³ with R¹², R¹³ = independently of one another alkyl radicals C₁-C₁₈ ;
• R⁷ is an alkyl radical with 2-15 C atoms, which is either linear or has a branch ≥ the 3-position (relative to the O function);
• R⁸ is an alkyl radical with 1-6 C atoms, which is either linear or has a branch ≥ the 3-position (relative to the O function);
• R⁹ is an alkyl radical with 2-15 C atoms, which is either linear or has a branch;
• n = an integer from 1 to 4,
• c + d ≤ 3 and c and d can assume values from 0 to 3; and wherein the content of aluminum compound Al(OCH₂R⁶)_{3-c-d}(OR⁷)_{b}[O(CHR⁸)ₙOR⁹]_{d}, based on the dissolved alkaline earth metal, is in the range from 0 to about 20 mol%, preferably from 0.2 and 15 mol%, particularly preferred from 0.5 and 4 mol%.

6. The solutions according to claim 1 to 5, **characterized in that** as an alkoxy function-containing alcohol HO(CHR⁸)ₙOR⁹ preferably is used a C₂-C₄ glycol monoether, for example 2-ethoxyethanol, 3-ethoxy-1-propanol, 3-ethoxy-1-butanol, 2-(2-ethylhexoxy) ethanol, 2-butoxyethanol, 2-hexyloxyethanol and 1,3-propylene glycol monobutyl ether or any mixture of at least two of the alcohols listed; as alcohol branched in the 2-position (HOCH₂R⁶) is used isobutanol, 2-methyl-1-pentanol, 2-ethyl-1-butanol, 2-ethyl-1-pentanol, 2-ethyl-4-methyl-1-pentanol, 2-propyl-1-heptanol, 2-methyl-1-hexanol, 2-ethylhexanol and 2-ethyl-5-methyl-1-octanol or any mixture of at least two of the alcohols listed, and as unbranched or one branch ≥ the 3-position containing primary alcohol with 2-15 carbon atoms (HOR⁷) is used ethanol, propanol, butanol, pentanol, hexanol, octanol, decanol, dodecanol, 3-methylbutan-1-ol or any mixture of at least two of the alcohols listed.

7. The solutions according to claim 1 to 6, **characterized in that** they contain 0.1 to 30 mol%, preferably 1 to 20 mol% and particularly preferred 3-15 mol% of the active base R₂Mg, determined by direct titration with sec-butanol and bichinoline as indicator and based on total alkaline earth metal M in solution, and that they are not pyrophoric according to the UN test.

8. A method for the preparation of alkaline earth alkoxide compounds M(OCH₂R⁶)_{2-a-b}(OR⁷)ₐ[O(CHR⁸)ₙOR⁹]_{b} in mixture with an alkaline earth metal alkyl compound M(R¹⁰R¹¹), both as defined in claim 1, **characterized in that** an alkaline earth metal or an alkaline earth metal mixture in an aprotic solvent is reacted with the alcohols and, after this reaction has ended, the solution of an alkaline earth metal alkyl compound M(R¹⁰R¹¹) is added, wherein the molar ratio of the alkaline earth metal oxide compound to the alkaline earth metal alkyl compound in the range from 99:1 to 70:30, particularly preferred from 95:5 to 80:20.

9. The method according to claim 8, **characterized in that** one or more alkyl aluminum compounds selected from trialkyl, alkyl alkoxy and/or alkyl halide compounds is added and the Al concentration, based on the dissolved alkaline earth metal, is in the range of 0 to about 20 mol%, preferably in the range from 0.2 to 15 mol%, particularly preferred from 0.5 to 4 mol%.

10. The method according to claim 8 or 9, **characterized in that** hydrocarbons are used as solvents, wherein either aliphatic solvents selected from the group consisting of cyclohexane, methylcyclohexane, hexane, heptane, octane, nonane, decane, dodecane, decalin and commercially available boiling cuts (gasoline fractions) or aromatic solvents selected from the group consisting of benzene, toluene, ethylbenzene, xylenes and cumene are used.

11. The method according to claim 8 to 10, **characterized in that** the reaction takes place at temperatures between about 0 and 180°C, preferably between about 40 and 140°C, in particular at the boiling point of the solvent.

12. The method according to claim 8 to 11, **characterized in that** as an alkoxy function-containing alcohol HO(CHR⁸)ₙOR⁹ preferably is used a C₂-C₄ glycol monoether, for example 2-ethoxyethanol, 3-ethoxy-1-propanol, 3-ethoxy-1-butanol, 2-(2-ethylhexoxy) ethanol, 2-butoxyethanol, 2-hexyloxyethanol and 1,3-propylene glycol monobutyl ether or any mixture of at least two of the alcohols listed; as alcohol branched in the 2-position (HOCH₂R⁶) is used isobutanol, 2-methyl-1-pentanol, 2-ethyl-1-butanol, 2-ethyl-1-pentanol, 2-ethyl-4-methyl-1-pentanol, 2-propyl-1-heptanol, 2-methyl-1-hexanol, 2-ethylhexanol and 2-ethyl-5-methyl-1-octanol or any mixture of at least two of the alcohols listed, and as unbranched or one branch ≥ the 3-position containing primary alcohol with 2-15 carbon atoms (HOR⁷) is used ethanol, propanol, butanol, pentanol, hexanol, octanol, decanol, dodecanol, 3-methylbutan-1-ol or any mixture of at least two of the alcohols listed.

13. A process for the preparation of alkaline earth alkoxide compounds M(OCH₂R⁶)_{2-a-b}(OR⁷)ₐ[O(CHR⁸)ₙOR⁹]_{b} in mixture with an alkaline earth metal alkyl compound M (R¹⁰R¹¹), both as defined in claim 1, **characterized in that** the solution of a metal alkyl compound M(R¹⁰R¹¹) in an aprotic solvent is mixed with the alcohols HOCH₂R⁶, HOR⁷ and/or HO(CHR⁸)ₙOR⁹, with half the molar ratio of the total mol of all alcohols to the alkaline earth metal alkyl compound in the range from 99:1 to 70:30, particularly preferred from 95: 5 to 80:20.

14. Use of solutions of alkaline earth alkoxide compounds M(OCH₂R⁶)_{2-a-b}(OR⁷)ₐ[O(CHR⁸)ₙOR⁹]_{b} in mixture with an alkaline earth metal alkyl compound M(R¹⁰R¹¹) according to claim 1 for the production of polymerization catalysts, in particular heterogenized polyolefin catalysts of the Ziegler -Natta type.

15. Use of solutions of alkaline earth alkoxide compounds M(OCH₂R⁶)_{2-a-b}(OR⁷)ₐ[O(CHR⁸)ₙOR⁹]_{b} in mixture with an alkaline earth metal alkyl compound M(R¹⁰R¹¹) according to claim 1 in organic synthesis, for example as a base.

## Revendications

1. Solutions de composés alcoolates de métaux alcalino-terreux M(OCH₂R⁶)_{2-a-b}(OR⁷)ₐ[O(CHR⁸)ₙOR⁹]_{b} en mélange avec un composé alkyl-métal alcalino-terreux M(R¹⁰R¹¹) à teneur en métal alcalino-terreux dans la plage de 0,2 à 1,8 mmole/g dans des solvants aprotiques, dans lesquelles
• M est un métal alcalino-terreux choisi parmi Mg, Ca, Ba, Sr ;
• OCH₂R⁶ est un radical alcoolate constitué d'au moins 3 et d'au maximum 40 atomes de carbone, ayant une ramification en position 2 par rapport à la fonction O, à savoir R⁶ = -CHR¹²R¹³ où R¹², R¹³ représentent indépendamment l'un de l'autre des radicaux alkyle en C₁-C₁₈;
• R⁷ est un radical alkyle ayant de 2 à 15 atomes de carbone, qui soit est linéaire soit comporte une ramification en ≥ de la position 3 (par rapport à la fonction O);
• R⁸ est un radical alkyle ayant de 1 à 6 atomes de carbone, qui soit est linéaire soit comporte une ramification en ≥ de la position 3 (par rapport à la fonction O) ;
• R⁹ est un radical alkyle ayant de 2 à 15 atomes de carbone, qui soit est linéaire soit comporte une ramification ;
• R¹⁰ et R¹¹ sont des radicaux alkyle quelconques ayant de 1 à 15 atomes de carbone,
• n est un nombre entier valant de 1 à 4,
• a + b est ≤ 2, a et b pouvant prendre des valeurs de 0 à 2 et
• le rapport molaire M(OCH₂R⁶)_{2-a-b}(OR⁷)ₐ[O(CHR⁸)ₙOR⁹]_{b} à M(R¹⁰R¹¹) se situe dans la plage de 99:1 à 70:30.

2. Solutions selon la revendication 1, **caractérisées en ce que** la teneur en métal alcalino-terreux se situe de préférence dans la plage de 0,4 à 1,6 mmole/g, de façon particulièrement préférée de 0,7 à 1,4 mmole/g.

3. Solutions selon la revendication 1 ou 2, **caractérisées en ce que** les viscosités à la température ambiante, à des concentrations de Mg de ≥ 1 mmole/g à ≤ 1,6 mmole/g se situent à ≤ 300 cP, de préférence ≤ 200 cP, de façon particulièrement préférée ≤ 100 cP.

4. Solutions selon la revendication 1 à la revendication 3, **caractérisées en ce que** le rapport molaire M(OCH₂R⁶)_{2-a-b}(OR⁷)ₐ[O(CHR⁸)ₙOR⁹]_{b} à M(R¹⁰R¹¹) se situe dans la plage de 95:5 à 80:20.

5. Solutions selon la revendication 1 à la revendication 4, **caractérisées en ce qu'**est contenu un composé d'aluminium Al(OCH₂R⁶)_{3-c-d}(OR⁷)_{c}[O(CHR⁸)ₙOR⁹]_{d}, dans lequel
• OCH₂R⁶ est un radical alcoolate constitué d'au moins 3 et d'au maximum 40 atomes de carbone ayant une ramification en position 2 par rapport à la fonction O, à savoir R⁶ = -CHR¹²R¹³ ou R¹², R¹³ représentent indépendamment l'un de l'autre des radicaux alkyle en C₁-C₁₈;
• R⁷ est un radical alkyle ayant de 2 à 15 atomes de carbone, qui soit est linéaire soit comporte une ramification en ≥ de la position 3 (par rapport à la fonction O) ;
• R⁸ est un radical alkyle ayant de 1 à 6 atomes de carbone, qui soit est linéaire soit comporte une ramification en ≥ de la position 3 (par rapport à la fonction O) ;
• R⁹ est un radical alkyle ayant de 2 à 15 atomes de carbone, qui soit est linéaire soit comporte une ramification ;
• n est un nombre entier valant de 1 à 4,
• c + d est ≤ 3, et c ainsi que d peuvent prendre des valeurs de 0 à 3 et la teneur en composé d'aluminium Al(OCH₂R⁶)_{3-c-d}(OR⁷)_{c}[O(CHR⁸)ₙOR⁹]_{d} par rapport au métal alcalino-terreux dissous se situe dans la plage de 0 à environ 20 % en moles, de préférence de 0,2 à 15 % en moles, de façon particulièrement préférée de 0,5 à 4 % en moles.

6. Solutions selon la revendication 1 à la revendication 5, **caractérisées en ce que** sont utilisés en tant qu'alcool HO(CHR⁸)ₙOR⁹ contenant une fonction alcoxy de préférence un monoéther de glycol en C₂-C₄, par exemple le 2-éthoxyéthanol, le 3-éthoxy-1-propanol, le 3-éthoxy-1-butanol, le 2-(2-éthylhexoxy)éthanol, le 2-butoxyéthanol, le 2-hexyloxyéthanol ainsi que l'éther monobutylique de 1,3-propylèneglycol ou un mélange quelconque d'au moins deux des alcools indiqués ; en tant qu'alcool ramifié en position 2 (HOCH₂R⁶) l'isobutanol, le 2-méthyl-1-pentanol, le 2-éthyl-1-butanol, le 2-éthyl-1-pentanol, le 2-éthyl-4-méthyl-1-pentanol, le 2-propyl-1-heptanol, le 2-méthyl-1-hexanol, le 2-éthylhexanol et le 2-éthyl-5-méthyl-1-octanol ou un mélange quelconque d'au moins deux des alcools indiqués et en tant qu'alcool primaire ayant de 2 à 15 atomes de carbone (HOR⁷), non ramifié ou comportant une ramification en ≥ de la position 3, l'éthanol, le propanol, le butanol, le pentanol, l'hexanol, l'octanol, le décanol, le dodécanol, le 3-méthylbutan-1-ol ou un mélange quelconque d'au moins deux des alcools indiqués.

7. Solutions selon la revendication 1 à la revendication 6, **caractérisées en ce qu'**elles contiennent de 0,1 à 30 % en moles, de préférence 1 à 20 % en moles et de façon particulièrement préférée 3-15 % en moles de base active R₂Mg, déterminé par titrage direct avec du sec-butanol et de la biquinoléine en tant qu'indicateur et par rapport au métal alcalino-terreux M présent au total en solution et ce qu'elles ne sont pas pyrophores selon le test UN.

8. Procédé pour la préparation de composés alcoolates de métaux alcalino-terreux M(OCH₂R⁶)_{2-a-b}(OR⁷)ₐ[O(CHR⁸)ₙOR⁹]_{b} en mélange avec un composé alkyl-métal alcalino-terreux M(R¹⁰R¹¹), les premiers et le dernier étant tels que définis dans la revendication 1, **caractérisé en ce qu'**un métal alcalino-terreux ou un mélange de métaux alcalino-terreux est mis en réaction avec les alcools dans un solvant aprotique et une fois cette réaction terminée est ajouté à la solution un composé alkyl-métal alcalino-terreux M(R¹⁰R¹¹), le rapport molaire du composé alcoolate de métal alcalino-terreux au composé alkyl-métal alcalino-terreux se situant dans la plage de 99:1 à 70:30, de façon particulièrement préférée de 95:5 à 80:20.

9. Procédé selon la revendication 8, **caractérisé en ce qu'**un ou plusieurs composé(s) alkylaluminium choisi(s) parmi un composé trialkyle, un composé alkylalcoxy et/ou un composé halogénure d'alkyle est/sont ajouté(s) et la concentration d'Al par rapport au métal alcalino-terreux dissous se situe dans la plage de 0 à environ 20 % en moles, de préférence dans la plage de 0,2 à 15 % en moles, de façon particulièrement préférée de 0,5 à 4 % en moles.

10. Procédé selon la revendication 8 ou 9, **caractérisé en ce que** sont utilisés comme solvant des hydrocarbures, étant utilisés soit des solvants aliphatiques choisis dans l'ensemble constitué par le cyclohexane, le méthylcyclohexane, l'hexane, l'heptane, l'octane, le nonane, le décane, le dodécane, la décaline ainsi que des coupes d'ébullition (fractions d'essence) du commerce, soit des solvants aromatiques choisis dans l'ensemble constitué par le benzène, le toluène, l'éthylbenzène, les xylènes ainsi que le cumène.

11. Procédé selon la revendication 8 à la revendication 10, **caractérisé en ce que** la réaction s'effectue à de températures comprises entre 0 et 180 °C, de préférence entre environ 40 et 140 °C, en particulier à la température d'ébullition du solvant.

12. Procédé selon la revendication 8 à la revendication 11, **caractérisé en ce que** sont utilisés en tant qu'alcool HO(CHR⁸)ₙOR⁹ contenant une fonction alcoxy de préférence un monoéther de glycol en C₂-C₄, par exemple le 2-éthoxyéthanol, le 3-éthoxy-1-propanol, le 3-éthoxy-1-butanol, le 2-(2-éthylhexoxy)éthanol, le 2-butoxyéthanol, le 2-hexyloxyéthanol ainsi que l'éther monobutylique de 1,3-propylèneglycol ou un mélange quelconque d'au moins deux des alcools indiqués ; en tant qu'alcool ramifié en position 2 (HOCH₂R⁶) l'isobutanol, le 2-méthyl-1-pentanol, le 2-éthyl-1-butanol, le 2-éthyl-1-pentanol, le 2-éthyl-4-méthyl-1-pentanol, le 2-propyl-1-heptanol, le 2-méthyl-1-hexanol, le 2-éthylhexanol et le 2-éthyl-5-méthyl-1-octanol ou un mélange quelconque d'au moins deux des alcools indiqués et en tant qu'alcool primaire ayant de 2 à 15 atomes de carbone (HOR⁷), non ramifié ou comportant une ramification en ≥ de la position 3, l'éthanol, le propanol, le butanol, le pentanol, l'hexanol, l'octanol, le décanol, le dodécanol, le 3-méthylbutan-1-ol ou un mélange quelconque d'au moins deux des alcools indiqués.

13. Procédé pour la préparation de composés alcoolates de métaux alcalino-terreux M(OCH₂R⁶)_{2-a-b}(OR⁷)ₐ[O(CHR⁸)ₙOR⁹]_{b} en mélange avec un composé alkyl-métal alcalino-terreux M(R¹⁰R¹¹), les premiers et le dernier étant tels que définis dans la revendication 1, **caractérisé en ce qu'**on ajoute à la solution d'un composé alkyl-métal M(R¹⁰R¹¹) dans un solvant aprotique les alcools HOCH₂R⁶, HOR⁷ et/ou HO(CHR⁸)ₙOR⁹, le demi-rapport molaire de la somme des moles de tous les alcools au composé alkyl-métal alcalino-terreux se situant dans la plage de 99:1 à 70:30, de façon particulièrement préférée de 95:5 à 80:20.

14. Utilisation de solutions de composés alcoolates de métaux alcalino-terreux M(OCH₂R⁶)_{2-a-b}(OR⁷)ₐ[O(CHR⁸)ₙOR⁹]_{b} en mélange avec un composé alkyl-métal alcalino-terreux M(R¹⁰R¹¹) selon la revendication 1 pour la production de catalyseurs de polymérisation, en particulier de catalyseurs polyoléfiniques hétérogénéisés du type Ziegler-Natta.

15. Utilisation de solutions de composés alcoolates de métaux alcalino-terreux M(OCH₂R⁶)_{2-a-b}(OR⁷)ₐ[O(CHR⁸)ₙOR⁹]_{b} en mélange avec un composé alkyl-métal alcalino-terreux M(R¹⁰R¹¹) selon la revendication 1 dans la synthèse organique, par exemple en tant que base.
